# FASCICULE DE BREVET EUROPEEN

(11) **EP 1 385 513 B1**
(45) Date de publication et mention de la délivrance du brevet: **06.09.2006**
(21) Numéro de dépôt: 02735502.3
(22) Date de dépôt: 26.04.2002
(51) Int. Cl.: A61K 31/437, A61P 35/00, A61K 45/06, A61K 31/435

(54) **ASSOCIATIONS PHARMACEUTIQUES A BASE DE DERIVES DE PYRIDOINDOLONE ET D'AGENTS ANTICANCEREUX**
PYRIDOINDOLONDERIVATE UND ANTIKREBSMITTEL ENTHALTENDE PHARMAZEUTISCHE MISCHUNGEN
PHARMACEUTICAL COMBINATIONS BASED ON PYRIDOINDOLONE DERIVATIVES AND ANTICANCER AGENTS

(30) Priorité: 27.04.2001 FR 0105843
(43) Date de publication de la demande: 04.02.2004
(73) Titulaire: Sanofi-Aventis, 75013 Paris (FR)
(72) Inventeur: BOURRIE, Bernard, F-34980 Saint Gely du Fesc (FR); CASELLAS, Pierre, F-34090 Montpellier (FR); DEROCQ, Jean-Marie, F-34570 Murviel les Montpellier (FR)
(74) Mandataire: Varady, Peter
(86) Numéro de dépôt international: PCT/FR2002/001450
(87) Numéro de publication internationale: WO 2002/087575

(56) Documents cités:
- FR-A- 2 003 999
- FR-A- 2 765 582
- US-A- 5 880 126

## Description

La présente invention a pour objet de nouvelles associations de dérivés de pyridoindolone avec des agents anticancéreux, et les compositions pharmaceutiques les contenant.

US-A-5 880 126 décrit des dérivés de pyridoindolone de structure différente qui peuvent être utilisés comme anti-inflammatoires et pour le traitement de la leucémie.

Les dérivés de pyridoindolone, utilisables dans la présente invention, sont les composés de formule : 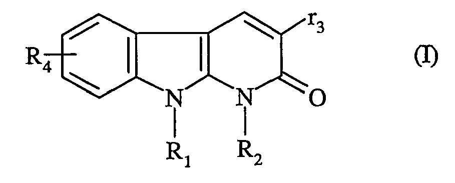 dans laquelle :
- R₁ représente un atome d'hydrogène ou un groupe méthyle ou éthyle ;
- R₂ représente un groupe méthyle ou éthyle ; ou bien
- R₁ et R₂ forment ensemble un groupe (CH₂)₃ ;
- r₃ représente, soit un groupe phényle éventuellement substitué par un atome d'halogène ou un groupe méthyle ou méthoxy, soit un groupe thiényle ;
- R₄ représente un atome d'hydrogène ou de chlore ou un groupe méthyle ou méthoxy.

Il a été trouvé que les composés de formule (I), agents anticancéreux, peuvent avantageusement être associés à d'autres agents anticancéreux. Des dérivés de pyridoindolone préférés sont les composés de formule : 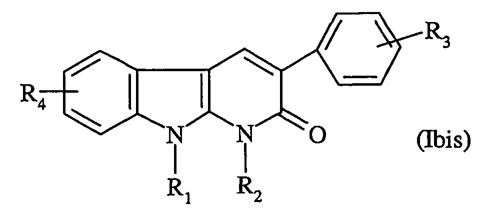 dans laquelle :
- R₁ représente un atome d'hydrogène, un groupe méthyle ou éthyle ;
- R₂ représente un groupe méthyle ou éthyle ; ou bien
- R₁ et R₂ forment ensemble un groupe (CH₂)₃ ;
- R₃ représente un atome d'hydrogène ou d'halogène ou un groupe méthyle ou méthoxy ;
- R₄ représente un atome d'hydrogène ou de chlore ou un groupe méthyle ou méthoxy.

Des dérivés de pyridoindolone particulièrement préférés sont les composés de formule: 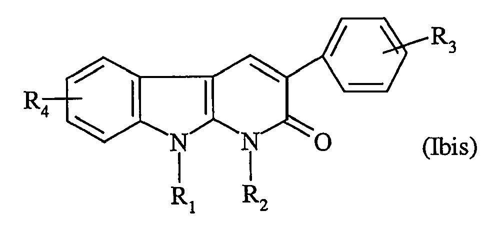 dans laquelle :
- R₁ représente un atome d'hydrogène, un groupe méthyle ou éthyle ;
- R₂ représente un groupe méthyle ou éthyle ;
- R₃ représente un atome d'hydrogène ou d'halogène ou un groupe méthyle ou méthoxy ;
- R₄ représente un atome d'hydrogène ou de chlore ou un groupe méthyle ou méthoxy.

Des dérivés de pyridoindolone plus particulièrement préférés sont les composés de formule: 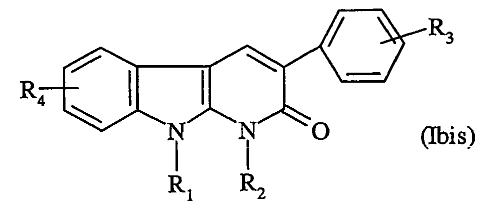 dans laquelle :
- R₁ représente un groupe méthyle ou éthyle ;
- R₂ représente un groupe méthyle ou éthyle ;
- R₃ représente un atome d'hydrogène ou d'halogène ou un groupe méthyle ou méthoxy ;
- R₄ représente un atome d'hydrogène ou de chlore ou un groupe méthyle ou méthoxy.
A titre d'exemple, des dérivés de pyridoindolone selon l'invention sont:
- la 6-chloro-1,9-diméthyl-3-phényl-1,9-dihydro-2H-pyrido[2,3-b]indol-2-one ;
   F = 178,5-179-5°C ;
- la 3-(4-methoxyphényl)-1,9-diméthyl-1,9-dihydro-2H-pyrido[2,3-b]indol-2-one ;
   F = 166-167°C ;
- la 1,6,9-triméthyl-3-(3-thiényl)-1,9-dihydro-2H-pyrido[2,3-b]indol-2-one ;
   RMN (200 MHz) : 2,6 ppm : s : 3H ; 4,1 ppm : s : 3H ; 4,2 ppm : s : 3H ; 7,1 ppm : d : 1H ; 7,4-7,9 ppm : m : 4H ; 8,3 ppm : d : 1H ; 8,7 ppm : s : 1H.
- la 1,6,9-triméthyl-3-phényl-1,9-dihydro-2H-pyrido[2,3-b]indol-2-one;
   F = 198-199°C ;
- la 1,6-diméthyl-3-phényl-1,9-dihydro-2H-pyrido[2,3-b]indol-2-one ;
   RMN (200 MHz) : 2,5 ppm : s : 3H ; 3,8 ppm : s : 3H ; 7,1 ppm : d : 1H ; 7,3-7,5 ppm : m : 4H ; 7,75 ppm : d : 2H ; 7,8 ppm : s : 1H ; 8,4 ppm : s : 1H ; 11,8 ppm : s : 1H.

Les composés de formule (I) sont préparés selon le procédé décrit dans le document FR 97 08409.

Les composés de formule (I) ont été testés *in vitro* sur une lignée cellulaire humaine de cancer de sein : la lignée MDA-MB-231 disponible auprès de l'American Type Culture Collection (référence HTB26).

L'évaluation de l'effet antiprolifératif est effectuée selon J.M. Derocq et al., FEBS Letters, 1998, 425, 419-425 : on mesure le taux d'incorporation de la [3H]thymidine dans l'ADN des cellules traitées, après 96 heures d'incubation d'un composé de formule (I). La concentration inhibitrice 50 (CI₅₀) est définie comme la concentration qui inhibe la prolifération cellulaire de 50 %.

Les composés de formule (I) présentent une CI₅₀ généralement inférieure à 10 µM sur la lignée MDA-MB-231.

Les composés de formule (I) ont également été testés sur une autre lignée cellulaire humaine de cancer du sein, dite lignée multi-résistante MDR, (de l'anglais multi-drug-resistant) et appelée MDA-A₁. Cette lignée est décrite par E. Collomb, C. Dussert et P.M. Martin dans Cytometry, 1991, 12(1), 15-25.

Le terme "multi-résistant" qui qualifie cette lignée, signifie que ladite lignée est peu sensible d'une manière générale aux drogues de chimiothérapie communément utilisées et en particulier aux antimitotiques d'origine naturelle tels que le paclitaxel, la vincristine, la vinblastine.

Les composés de formule (I) présentent une CI₅₀ généralement inférieure à 10 µM sur la lignée multi-résistante MDA-A₁.

Ainsi les composés de formule (I) inhibent la prolifération des cellules tumorales y compris la prolifération des cellules présentant une multi-résistance.

Plusieurs composés de formule (I) ont également été évalués *in vivo* sur un modèle de xénogreffe de tumeurs humaines implantées en sous-cutané sur la souris immuno-déprimée SCID (de l'anglais : Severe Combined Immuno Deficiency).

Le traitement des animaux avec un composé de formule (I) débute 6 à 7 jours après l'implantation, lorsque la tumeur atteint une masse tumorale d'environ 60 mg. Le composé, en solution dans un solvant, est alors administré par voie orale.

L'activité antitumorale est évaluée lorsque la masse tumorale moyenne atteint environ 1000 mg chez les animaux contrôles, traités avec le solvant uniquement : on mesure le rapport T/C, T représentant le poids moyen des tumeurs chez les animaux traités et C représentant le poids moyen des tumeurs chez les animaux contrôles. Un rapport T/C inférieur ou égal à 42 % est considéré comme indicateur d'une activité antitumorale significative selon Stuart T et al., dans J. Med. Chem., 2001, 44 (11), 1758-1776. Pour une dose journalière administrée comprise entre 50 et 300 mg/kg, certains composés de formule (I) ont conduit à un rapport T/C inférieur à 20 %.

Les effets antiprolifératifs de l'association d'un composé de formule (I) avec un autre agent anticancéreux ont été mesurés sur la lignée MDA-A₁, dite lignée multi-résistante citée ci-dessus, selon la technique référencée (J.M. Derocq et al.). La CI₅₀ obtenue pour l'agent anticancéreux seul a été comparée à celle obtenue pour l'association du même agent anticancéreux avec un composé de formule (I). Une nette diminution de la CI₅₀ a été observée. Les CI₅₀ peuvent être divisées par un facteur allant de 2 à 100 et même au-delà de 100 pour certains dérivés de pyridoindolone de formule (I) associés à un autre agent anticancéreux.

A titre d'exemple, la CI₅₀ obtenue pour le paclitaxel seul a été comparée à celle obtenue pour l'association du paclitaxel avec la 1,6,9-triméthyl-3-phényl-1,9-dihydro-2*H*-pyrido[2,3-b]indol-2-one. La CI₅₀ est divisée par un facteur allant de 14 à 100 suivant les concentrations de 1,6,9-triméthyl-3-phényl-1,9-dihydro-2*H*-pyrido[2,3-b]indol-2-one employées.

Les résultats des essais montrent un effet synergique et une potentialisation des effets antiprolifératifs des composants des associations de l'invention.

Selon la présente invention, le ou les composés de formule (I) sont administrés en association avec un (ou plusieurs) principe(s) actif(s) anticancéreux. Ces principes actifs peuvent être notamment des composés antitumoraux tels que les agents alkylants, les alkylsulfonates (busulfan), la dacarbazine, la procarbazine, les moutardes azotées (chlorméthine, melphalan, chlorambucil), cyclophosphamide, ifosfamide ; les nitrosourées tels que la carmustine, la lomustine, la sémustine, la streptozocine ; les alcaloïdes antinéoplasiques tels que la vincristine, la vinblastine ; les taxanes tel que le paclitaxel ; les antibiotiques antinéoplasiques tels que l'actinomycine ; les agents intercalants, les antimétabolites antinéoplasiques, les antagonistes des folates, le méthotrexate ; les inhibiteurs de la synthèse des purines ; les analogues de la purine tels que mercaptopurine, 6-thioguanine ; les inhibiteurs de la synthèse des pyrimidines, les inhibiteurs d'aromatase, la capécitabine, les analogues de la pyrimidine tels que fluorouracil, gemcitabine, cytarabine et cytosine arabinoside ; le bréquinar ; les agonistes et antagonistes hormonaux anticancéreux incluant le tamoxifène ; les inhibiteurs de kinase, l'imatinib ; les inhibiteurs de facteurs de croissance ; certains anti-inflammatoires agissant dans le domaine de la cancérologie tels que le pentosane polysulfate, les corticostéroïdes, la prednisone, la dexamethasone ; les antitopoisomérases tels que l'étoposide, les antracyclines incluant la doxorubicine, la bléomycine, la mitomycine et la méthramycine ; les complexes métalliques anticancéreux, les complexes du platine, le cisplatine, le carboplatine, l'oxaliplatine ; l'interféron alpha, le triphénylthiophosphoramide, l'altrétamine ; les agents antiangiogéniques ; les adjuvants d'immunothérapie.

Les associations de l'invention sont utiles pour la prévention et le traitement des maladies causées ou exacerbées par la prolifération des cellules tumorales, telles que les tumeurs primaires ou métastasiques, des carcinomes et cancers, en particulier : cancer du sein ; cancer du poumon ; cancer de l'intestin grêle, cancer du colon et du rectum ; cancer des voies respiratoires, de l'oropharynx et de l'hypopharynx ; cancer de l'oesophage ; cancer du foie, cancer de l'estomac, cancer des canaux biliaires, cancer de la vésicule biliaire, cancer du pancréas ; cancers des voies urinaires y compris rein et urothelium, cancer de la vessie ; cancers du tractus génital féminin y compris cancer de l'utérus, du col de l'utérus, des ovaires, chloriocarcinome et trophoblastome ; cancers du tractus génital masculin y compris cancer de la prostate, des vésicules séminales, des testicules, tumeurs des cellules germinales ; cancers des glandes endocrines y compris cancer de la thyroïde, de l'hypophyse, des glandes surrénales ; cancers de la peau y compris hémangiomes, mélanomes, sarcomes, incluant le sarcome de Kaposi ; tumeurs du cerveau, des nerfs, des yeux, des méninges, incluant astrocytomes, gliomes, glioblastomes, rétinoblastomes, neurinomes, neuroblastomes, schwannomes, méningiomes ; tumeurs venant de tumeurs malignes hématopoïétiques incluant leucémies, chloromes, plasmacytomes, mycosis fongoïde, lymphome ou leucémie des cellules T, lymphome non hodgkinien, hémopathies malignes, myélomes.

La présente invention a également pour objet des compositions pharmaceutiques contenant, en tant que principe(s) actif(s), une quantité thérapeutiquement efficace d'au moins un composé de formule (I) ou un sel pharmaceutiquement acceptable, un hydrate ou un solvat dudit composé, et une quantité thérapeutiquement efficace d'un (ou plusieurs) autre(s) principe(s) actif(s) anticancéreux, ainsi qu'un ou plusieurs excipients pharmaceutiquement acceptables.

Lesdits excipients sont choisis selon la forme pharmaceutique et le mode d'administration souhaité, parmi les excipients habituels qui sont connus dans l'art antérieur.

Les compositions pharmaceutiques de la présente invention peuvent être préparées pour l'administration orale, sublinguale, sous-cutanée, intramusculaire, intraveineuse, topique, locale, intratrachéale, intranasale, transdermique ou rectale, ou toute autre voie adéquate, aux animaux et aux êtres humains pour la prévention ou le traitement des maladies ci-dessus.

Les composés de formule (I) ci-dessus peuvent être utilisés à des doses journalières de 0,002 à 2000 mg par kilogramme de poids corporel du mammifère à traiter, de préférence à des doses journalières de 0,1 à 300 mg/kg. Chez l'être humain, la dose peut varier de préférence de 0,02 à 10000 mg par jour, plus particulièrement de 1 à 3000 mg, selon l'âge du sujet à traiter ou le type de traitement (prophylactique ou curatif).

Les principes actifs anticancéreux, avec lesquels les composés de formule (I) sont associés, sont utilisés aux doses habituelles.

Les formes d'administration appropriées comprennent les formes orales telles que les comprimés, les gélules molles ou dures, les poudres, les granules et les solutions ou suspensions orales, les formes pour administration sublinguale, buccale, intratrachéale, intraoculaire, intranasale, par inhalation, les formes pour administration topique, transdermique, sous-cutanée, intramusculaire ou intraveineuse, les formes pour administration rectale et les implants. Pour l'application topique, on peut utiliser les associations selon l'invention dans des crèmes, gels, pommades ou lotions.

Selon la pratique habituelle, le dosage approprié à chaque patient est déterminé par le médecin selon le mode d'administration, l'âge, le poids et la réponse dudit patient.

Selon un autre aspect de l'invention, le ou les composés de formule (I) et un (ou plusieurs) autre(s) principe(s) actif(s) anticancéreux peuvent être administrés de manière simultanée, séquentielle ou étalée dans le temps, pour le traitement des maladies causées ou exacerbées par la prolifération des cellules tumorales.

Les associations de l'invention peuvent être présentées sous forme de kit contenant au moins un composé de formule (I), d'une part, et un (ou plusieurs) autre(s) principe(s) actif(s) anticancéreux, d'autre part.

Selon un autre de ses aspects, l'invention concerne aussi un traitement des maladies causées ou exacerbées par la prolifération des cellules tumorales qui consiste à administrer à un sujet en ayant besoin une quantité thérapeutiquement efficace d'au moins un composé de formule (I) en association avec un (ou plusieurs) autre(s) principe(s) actif(s) anticancéreux.

## Revendications

1. Association d'au moins un composé de formule : 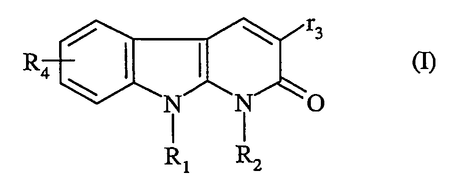 dans laquelle :
- R₁ représente un atome d'hydrogène ou un groupe méthyle ou éthyle ;
- R₂ représente un groupe méthyle ou éthyle ; ou bien
- R₁ et R₂ forment ensemble un groupe (CH₂)₃ ;
- r₃ représente, soit un groupe phényle éventuellement substitué par un atome d'halogène ou un groupe méthyle ou méthoxy, soit un groupe thiényle ;
- R₄ représente un atome d'hydrogène ou de chlore ou un groupe méthyle ou méthoxy ;
avec un (ou plusieurs) principe(s) actif(s) anticancéreux.

2. Association selon la revendication 1 d'au moins un composé de formule : 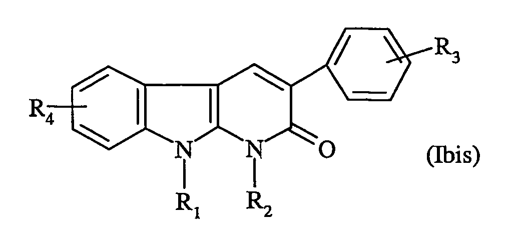 dans laquelle :
- R₁ représente un atome d'hydrogène, un groupe méthyle ou éthyle ;
- R₂ représente un groupe méthyle ou éthyle ; ou bien
- R₁ et R₂ forment ensemble un groupe (CH₂)₃ ;
- R₃ représente un atome d'hydrogène ou d'halogène ou un groupe méthyle ou méthoxy ;
- R₄ représente un atome d'hydrogène ou de chlore ou un groupe méthyle ou méthoxy.

3. Association selon la revendication 1 ou la revendication 2 d'au moins un composé de formule : 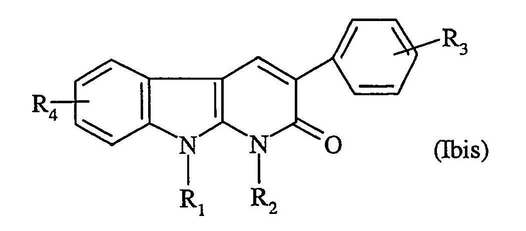 dans laquelle :
- R₁ représente un atome d'hydrogène, un groupe méthyle ou éthyle ;
- R₂ représente un groupe méthyle ou éthyle ;
- R₃ représente un atome d'hydrogène ou d'halogène ou un groupe méthyle ou méthoxy ;
- R₄ représente un atome d'hydrogène ou de chlore ou un groupe méthyle ou méthoxy.

4. Association selon la revendication 1 dans laquelle le composé de formule (I) est un des composés cités ci-après :
- la 6-chloro-1,9-diméthyl-3-phényl-1,9-dihydro-2H-pyrido[2,3-b]indol-2-one ;
- la 3-(4-methoxyphényl)-1,9-diméthyl-1,9-dihydro-2H-pyrido[2,3-b]indol-2-one ;
- la 1,6,9-triméthyl-3-(3-thiényl)-1,9-dihydro-2H-pyrido[2,3-b]indol-2-one ;
- la 1,6,9-triméthyl-3-phényl-1,9-dihydro-2H-pyrido[2,3-b]indol-2-one ;
- la 1,6-diméthyl-3-phényl-1,9-dihydro-2H-pyrido[2,3-b]indol-2-one.

5. Composition pharmaceutique comprenant au moins un composé de formule : 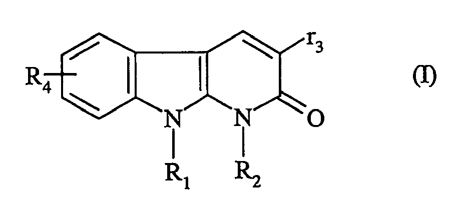 dans laquelle :
- R₁ représente un atome d'hydrogène ou un groupe méthyle ou éthyle ;
- R₂ représente un groupe méthyle ou éthyle ; ou bien
- R₁ et R₂ forment ensemble un groupe (CH₂)₃ ;
- r₃ représente, soit un groupe phényle éventuellement substitué par un atome d'halogène ou un groupe méthyle ou méthoxy, soit un groupe thiényle ;
- R₄ représente un atome d'hydrogène ou de chlore ou un groupe méthyle ou méthoxy ;
en association avec un (ou plusieurs) principe(s) actif(s) anticancéreux et un ou plusieurs excipients pharmaceutiquement acceptables.

6. Kit pour le traitement de la prolifération des cellules tumorales contenant d'une part au moins un composé de formule : 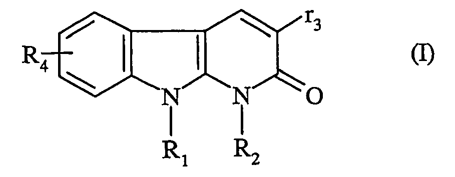 dans laquelle :
- R₁ représente un atome d'hydrogène ou un groupe méthyle ou éthyle ;
- R₂ représente un groupe méthyle ou éthyle ; ou bien
- R₁ et R₂ forment ensemble un groupe (CH₂)₃ ;
- r₃ représente, soit un groupe phényle éventuellement substitué par un atome d'halogène ou un groupe méthyle ou méthoxy, soit un groupe thiényle ;
- R₄ représente un atome d'hydrogène ou de chlore ou un groupe méthyle ou méthoxy ;
d'autre part un (ou plusieurs) principe(s) actif(s) anticancéreux ;
ces composés étant dans des compartiments distincts et étant destinés à être administrés de façon simultanée, séquentielle ou étalée dans le temps.

## Claims

1. Combination of at least one compound of formula: 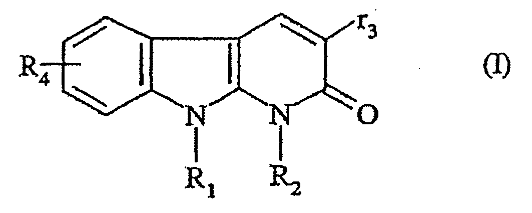 in which:
- R₁ represents a hydrogen atom or a methyl or ethyl group;
- R₂ represents a methyl or ethyl group; or
- R₁ and R₂ together form a (CH₂)₃ group;
- r₃ represents either a phenyl group optionally substituted with a halogen atom or a methyl or methoxy group, or a thienyl group;
- R₄ represents a hydrogen or chlorine atom or a methyl or methoxy group;
with one (or more) anticancer active principle(s).

2. The combination according to Claim 1, of at least one compound of formula: 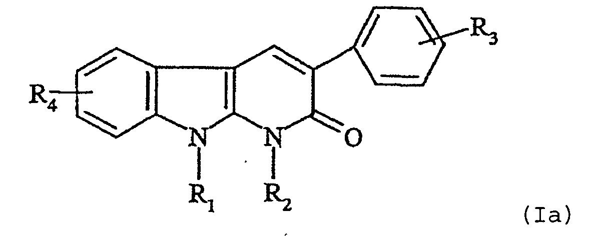 in which:
- R₁ represents a hydrogen atom or a methyl or ethyl group;
- R₂ represents a methyl or ethyl group; or
- R₁ and R₂ together form a (CH₂)₃ group;
- R₃ represents a hydrogen or halogen atom or a methyl or methoxy group;
- R₄ represents a hydrogen or chlorine atom or a methyl or methoxy group.

3. Combination according to Claim 1 or Claim 2, of at least one compound of formula: 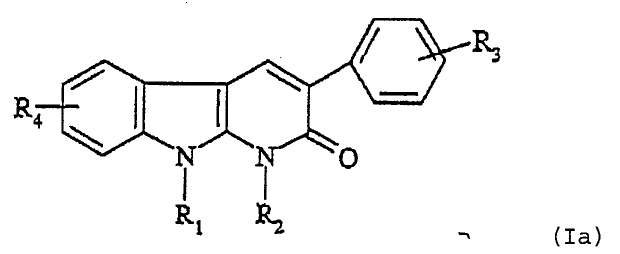 in which:
- R₁ represents a hydrogen atom or a methyl or ethyl group;
- R₂ represents a methyl or ethyl group;
- R₃ represents a hydrogen or halogen atom or a methyl or methoxy group;
- R₄ represents a hydrogen or chlorine atom or a methyl or methoxy group.

4. Combination according to Claim 1, in which the compound of formula (I) is one of the compounds mentioned below:
- 6-chloro-1,9-dimethyl-3-phenyl-1,9-dihydro-2H-pyrido[2,3-b]indol-2-one;
- 3-(4-methoxyphenyl)-1,9-dimethyl-1,9-dihydro-2H-pyrido[2,3-b]indol-2-one;
- 1,6,9-trimethyl-3-(3-thienyl)-1,9-dihydro-2H-pyrido[2,3-b]indol-2-one;
- 1,6,9-trimethyl-3-phenyl-1,9-dihydro-2H-pyrido[2,3-b]indol-2-one;
- 1,6-dimethyl-3-phenyl-1,9-dihydro-2H-pyrido[2,3-b]indol-2-one.

5. Pharmaceutical composition comprising at least one compound of formula: 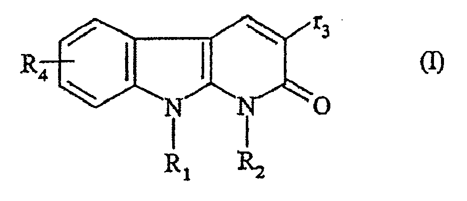 in which:
- R₁ represents a hydrogen atom or a methyl or ethyl group;
- R₂ represents a methyl or ethyl group; or
- R₁ and R₂ together form a (CH₂)₃ group;
- r₃ represents either a phenyl group optionally substituted with a halogen atom or a methyl or methoxy group, or a thienyl group;
- R₄ represents a hydrogen or chlorine atom or a methyl or methoxy group;
in combination with one (or more) anticancer active principle(s) and one or more pharmaceutically acceptable excipients.

6. Kit for treating the proliferation of tumour cells, containing, firstly, at least one compound of formula: 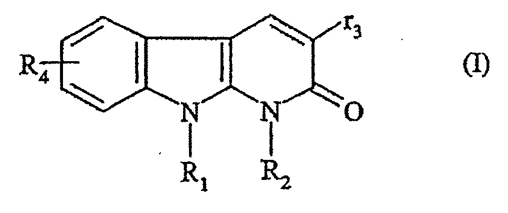 in which:
- R₁ represents a hydrogen atom or a methyl or ethyl group;
- R₂ represents a methyl or ethyl group; or
- R₁ and R₂ together form a (CH₂)₃ group;
- r₃ represents either a phenyl group optionally substituted with a halogen atom or a methyl or methoxy group, or a thienyl group;
- R₄ represents a hydrogen or chlorine atom or a methyl or methoxy group;
and, secondly, one (or more) anticancer active principle(s);
these compounds being in separate compartments and being intended to be administered simultaneously, sequentially or separately over time.

## Patentansprüche

1. Kombination von mindestens einer Verbindung der Formel: 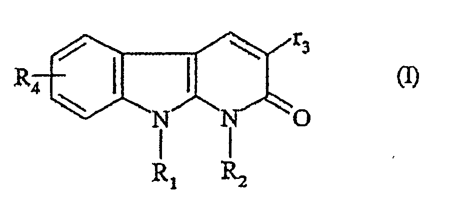 worin:
- R₁ für ein Wasserstoffatom oder eine Methyl- oder Ethylgruppe steht;
- R₂ für eine Methyl- oder Ethylgruppe steht oder
- R₁ und R₂ gemeinsam eine (CH₂)₃-Gruppe bilden;
- r₃ entweder für eine gegebenenfalls durch ein Halogenatom oder eine Methyl- oder Methoxygruppe substituierte Phenylgruppe oder eine Thienylgruppe steht;
- R₄ für ein Wasserstoff- oder Chloratom oder eine Methyl- oder Methoxygruppe steht;
mit einem (oder mehreren) Antikrebswirkstoff(en).

2. Kombination nach Anspruch 1 von mindestens einer Verbindung der Formel: 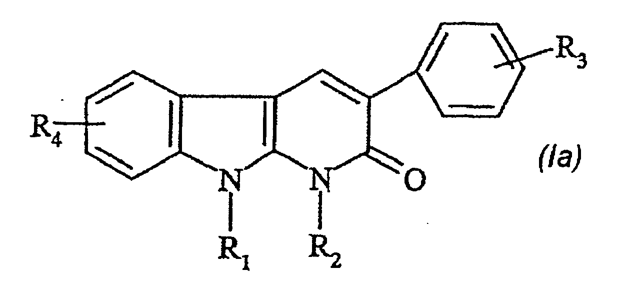 worin:
- R₁ für ein Wasserstoffatom oder eine Methyl- oder Ethylgruppe steht;
- R₂ für eine Methyl- oder Ethylgruppe steht oder
- R₁ und R₂ gemeinsam eine (CH₂)₃-Gruppe bilden;
- R₃ für ein Wasserstoff- oder Halogenatom oder eine Methyl- oder Methoxygruppe steht;
- R₄ für ein Wasserstoff- oder Chloratom oder eine Methyl- oder Methoxygruppe steht.

3. Kombination nach Anspruch 1 oder 2 von mindestens einer Verbindung der Formel: 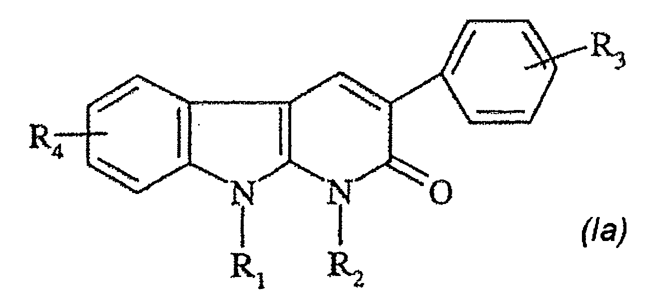 worin:
- R₁ für ein Wasserstoffatom oder eine Methyl- oder Ethylgruppe steht;
- R₂ für eine Methyl- oder Ethylgruppe steht;
- R₃ für ein Wasserstoff- oder Halogenatom oder eine Methyl- oder Methoxygruppe steht;
- R₄ für ein Wasserstoff- oder Chloratom oder eine Methyl- oder Methoxygruppe steht.

4. Kombination nach Anspruch 1, in der es sich bei der Verbindung der Formel (I) um eine der nachstehend aufgeführten Verbindungen handelt:
- 6-Chlor-1,9-dimethyl-3-phenyl-1,9-dihydro-2H-pyrido[2,3-b]indol-2-on;
- 3-(4-Methoxyphenyl)-1,9-dime-thyl-1,9-dihydro-2H-pyrido[2,3-b]indol-2-on;
- 1,6,9-Trimethyl-3-(3-thienyl)-1,9-dihydro-2H-pyrido[2,3-b]indol-2-on;
- 1,6,9-Trimethyl-3-phenyl-1,9-dihydro-2H-pyrido-[2,3-b]indol-2-on;
- 1,6-Dimethyl-3-phenyl-1,9-dihydro-2H-pyrido[2,3-b]indol-2-on.

5. Pharmazeutische Zusammensetzung, enthaltend mindestens eine Verbindung der Formel: 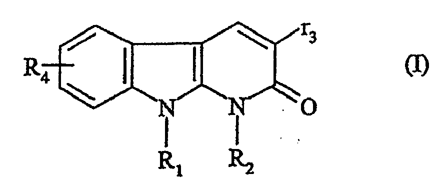 worin:
- R₁ für ein Wasserstoffatom oder eine Methyl- oder Ethylgruppe steht;
- R₂ für eine Methyl- oder Ethylgruppe steht oder
- R₁ und R₂ gemeinsam eine (CH₂)₃-Gruppe bilden;
- r₃ entweder für eine gegebenenfalls durch ein Halogenatom oder eine Methyl- oder Methoxygruppe substituierte Phenylgruppe oder eine Thienylgruppe steht;
- R₄ für ein Wasserstoff- oder Chloratom oder eine Methyl- oder Methoxygruppe steht;
in Kombination mit einem (oder mehreren) Antikrebswirkstoff(en) und einem oder mehreren pharmazeutisch unbedenklichen Hilfsstoffen.

6. Kit zur Behandlung der Proliferation von Tumorzellen, enthaltend zum einen mindestens eine Verbindung der Formel: 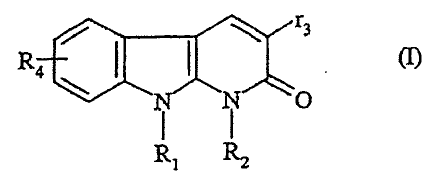 worin:
- R₁ für ein Wasserstoffatom oder eine Methyl- oder Ethylgruppe steht;
- R₂ für eine Methyl- oder Ethylgruppe steht oder
- R₁ und R₂ gemeinsam eine (CH₂)₃-Gruppe bilden;
- r₃ entweder für eine gegebenenfalls durch ein Halogenatom oder eine Methyl- oder Methoxygruppe substituierte Phenylgruppe oder eine Thienylgruppe steht;
- R₄ für ein Wasserstoff- oder Chloratom oder eine Methyl- oder Methoxygruppe steht;
und zum anderen eine (oder mehrere) Antikrebswirkstoff(e);
wobei diese Verbindungen in getrennten Kompartimenten vorliegen und zur gleichzeitigen, aufeinanderfolgenden oder zeitlich versetzten Verabreichung vorgesehen sind.
